# EUROPEAN PATENT SPECIFICATION

(11) **EP 1 890 687 B1**
(45) Date of publication and mention of the grant of the patent: **17.09.2008**
(21) Application number: 05749611.9
(22) Date of filing: 14.06.2005
(51) Int. Cl.: A61K 31/196, A61K 47/38, A61K 47/10

(54) **STABLE PHARMACEUTICAL GEL OF DICLOFENAC SODIUM**
STABILES PHARMAZEUTISCHES GEL AUS DICLOFENAC-NATRIUM
GEL PHARMACEUTIQUE STABLE DE DICLOFÉNAC SODIQUE

(43) Date of publication of application: 27.02.2008
(73) Proprietor: Uni-Pharma Kleon Tsetis Pharmaceutical Laboratories S.A., 145 64 Kifissia (GR)
(72) Inventor: TSETI, Ioulia, GR-145 61 Kifissia (GR)
(74) Representative: Patrinos-Kilimiris, Anna
(86) International application number: PCT/GR2005/000023
(87) International publication number: WO 2006/134406

(56) References cited:
- EP-A- 0 450 123
- EP-A- 0 524 582
- WO-A-00/44347
- WO-A-20/05027977
- US-A- 4 670 254
- US-A1- 2003 161 867

## Description

### TECHNICAL FIELD

The invention relates to a new formulation of Diclofenac Na gel for topical administration through the skin intended for the relief of pain and inflammation in various conditions: local symptomatic relief of pain and inflammation, musculoskeletal and joint disorders such as rheumatoid arthritis, osteoarthritis, and actinic keratosis, soft tissue disorders and other painful conditions. Martindale, The complete drug reference, 34th edition, pp. 32-34

### PRIOR ART

Stable semisolid preparations of Diclofenac Na (ointments/creams) are already being marketed, and there are several documents that disclose compositions for topical administration (gels, creams ointments etc.) that comprise variable concentrations of antiinflammatory agents. Within them, the following can be highlighted:
AU2003269266 - 2004-04-23, CA2496469 - 2004-03-04, HK1012570 - 2000-11-24, CN1249930 - 2000-04-12, CA2496469 - 2004-03-04, JP9012452-1997-01-14, US5350769 - 1994-09-27, JP3291222 - 1991-12-20, US4670254 - 1987-06-02,

### PRESENT INVENTION

The present invention is a device comprising a tube suitable for pharmaceutical use, like anyone of the tubes disclosed in other relevant documents containing the Diclofenac Na formulation with the suitable excipients as described below.

The present invention is not limited to the tubes mentioned herein, however, the tube of the present document is one of the various preferred tubes.

None of the prior art documents discloses the present invention, i.e. a Diclofenac Na pharmaceutical gel packaged in a blind tube intended for topical administration comprised of the same formulation.

Although other Diclofenac Na gels are referred in other patent documents nowhere in said documents the same formulation applies.

Thus, the present invention relating in a "gel" comprising "Diclofenac Na intended for topical administration" is new.

Patients found that the topical effect after the administration of Diclofenac Na gel according to the present invention was effective towards inflammatory diseases.

Additionally to this, in the permeation experiments conducted, it was found that an increased amount of Diclofenac Na was topically absorbed through the skin in comparison with two other commercially available gels containing the same active substance but in a different formulation.

Given the importance of an optimized absorption of Diclofenac Na through the skin, and according to in-vitro experiments that took place in the university of Athens-Greece, we believe that our invention shows a major advantage over the already existing formulations of the prior mentioned arts.

According to the present invention, the amount of Diclofenac Na is 10,0 mg per 1,0 gr of the formulation.

Although there is no essential upper limit for diclofenac sodium, a suitable maximum is 10% (w/w) in order to obtain the maximum advantage. Additionally there is no essential lower limit for diclofenac sodium, a suitable minimum is 0,3 % (w/w) in order to obtain the maximum advantage. Most preferably the gel of the invention comprises diclofenac sodium in a concentration of 1% (w/w) in order to obtain the maximum advantage.

Except from Diclofenac sodium which is the active substance, other pharmaceutically acceptable excipients are also included. The gel composition of the invention comprises, at least one, preferably more than one, most preferably -for obtaining the maximum advantage- all of the following compounds.
Propylene Glycol, Methocel E4 MP, Methyl-p-hydroxybenzoate, Methyl salicylate, Menthol and purified water for pharmaceutical use.

According to the present invention, the amounts of excipients may preferably be as follows:

| | |
|---|---|
| Propylene Glycol | 80,00 mg per 1,0 gr of the formulation |
| Methocel E4 MP | 20,00 mg per 1,0 gr of the formulation |
| Methyl-p-hydroxybenzoate | 1,50 mg per 1,0 gr of the formulation |
| Methyl salicylate | 0,020 mg per 1,0 gr of the formulation |
| Menthol | 0,030 mg per 1,0 gr of the formulation |
| Water for pharmaceutical use | q.s |

One skilled in the art would be able to select other pharmaceutically acceptable excipients for the composition of the invention. For example, the composition may comprise, additionally, any one or more of vehicles, fragrances and thickeners, which are pharmaceutically and cosmetically acceptable.

Moreover
a) the final preparation of the present invention can preferably -for obtaining the maximum advantage- comprise Propylene glycol in an amount of 30,0 mg to 150,0 mg per gram of the formulation.
b) the final preparation of the present invention can preferably -for obtaining the maximum advantage- comprise Methocel E4 (or any other Methocel derivative) in an amount of 10,0 mg per 40,0 mg per gram of the formulation.
c) the final preparation of the present invention can preferably -for obtaining the maximum advantage- comprise Methyl-p-hydroxybenzoate (or any other preservative) in an amount of 0,5 mg per 3,0 mg per gram of the formulation.
d) the final preparation of the present invention can preferably -for obtaining the maximum advantage- comprise Methyl salicylate in an amount of 0,005 mg per 0,050 mg per gram of the formulation.
e) the final preparation of the present invention can preferably -for obtaining the maximum advantage- comprise Menthol (or any other flavouring agent) in an amount of 0,010 mg per 0,060 per gram of the formulation.

### Method of production

The process described below is a non-limiting, illustrative method to produce the Diclofenac Na semisolid gel of the invention. Importantly, specific settings and parameters of the production process can be readily optimized by one of skill in the art in order to produce gels with particularly desired characteristics.

This illustrative process can be divided into 9 steps, as follows:
1) Propylene glycol (0,080 kg) is brought into a vessel made of stainless steel of suitable volume where it is being heated until the temperature reaches 50° C. In the same vessel, Methyl-p-hydroxybenzoate is added under continious stirring until producing a solution.
2) 0,06 kg of purified water for pharmaceutical use is then slowly added under stirring into the previously prepared solution for about 15 mins, and the produced solution is allowed to cool down at room temperature.
3) Next, the active substance, Diclofenac Na (0,010 kg) is added under stirring until a solution is produced.
4) The resulting solution is then checked regarding the desired pH value and is then fixed to a value of 5.50, using a buffer of Phosphoric acid if required.
5) The remaining indicated amount of purified water (0,289 kg) for pharmaceutical use is then added under stirring for a period of 10 mins,
6) The resulting solution is heated up to 40° C. After this temperature has been reached, Methocel E4 MP (0,020) is added in the same vessel under stirring which starts gently and becomes more intense later.
7) A less intense stirring follows and the resulting solution is left to cool to room temperature so that the polymeric material (Methocel) is fully hydrated.
8) The whole amount of Menthol (0,030 kg) is mixed with Propylene glycol (0,070 kg) and the mixture is being added to the previous solution of step 8 under stirring.
9) Water is being added if necessary to replace the amount of any potential water loss and the finally produced gel is immediately being filled to the containers.

### DESCRIPTION OF EXAMPLES AND PREFERRED EMBODIMENTS

Taking into account that the present invention is capable of being performed in many different embodiments, some of the preferred embodiments are described below. However, it must be understood that the present examples must be considered as showing the principles of the invention but they are not directed to limit the invention to the examples specifically illustrated.

### Example 1: Formulation of a gel of diclofenac sodium according to the present invention

In the following example (no 1) the semisolid formulation containing Diclofenac Na are identical with the composition of the pilot-batch gel products under the name Miniflam® (trade mark of UNI-PHARMA).

| Example 1: (Gel 1 %) | |
|---|---|
| Diclofenac Na | 10,00 mg per 1,0 gr of the formulation |
| Propylene Glycol | 80,00 mg per 1,0 gr of the formulation |
| Methocel E4 MP | 20,00 mg per 1,0 gr of the formulation |
| Methyl-p-hydroxybenzoate | 1,50 mg per 1,0 gr of the formulation |
| Methyl salicylate | 0,020 mg per 1,0 gr of the formulation |
| Menthol | 0,030 mg per 1,0 gr of the formulation |
| Water for pharmaceutical use | q.s |
| | |

| Example 2: (Gel 1%) | |
|---|---|
| Diclofenac Na | 10,00 mg per 1,0 gr of the formulation |
| Propylene Glycol | 70,00 mg per 1,0 gr of the formulation |
| Methocel E4 MP | 30,00 mg per 1,0 gr of the formulation |
| Methyl-p-hydroxybenzoate | 1,50 mg per 1,0 gr of the formulation |
| Methyl salicylate | 0,020 mg per 1,0 gr of the formulation |
| Menthol | 0,030 mg per 1,0 gr of the formulation |
| Water for pharmaceutical use | q.s |
| | |

| Example 3: (Gel 0,5%) | |
|---|---|
| Diclofenac Na | 5,00 mg per 1,0 gr of the formulation |
| Propylene Glycol | 70,00 mg per 1,0 gr of the formulation |
| Methocel E4 MP | 30,00 mg per 1,0 gr of the formulation |
| Methyl-p-hydroxybenzoate | 1,50 mg per 1,0 gr of the formulation |
| Methyl salicylate | 0,020 mg per 1,0 gr of the formulation |
| Menthol | 0,030 mg per 1,0 gr of the formulation |
| Water for pharmaceutical use | q.s |
| | |

| Example 4: (Gel 0,5%) | |
|---|---|
| Diclofenac Na | 5,00 mg per 1,0 gr of the formulation |
| Propylene Glycol | 80,00 mg per 1,0 gr of the formulation |
| Methocel E4 MP | 20,00 mg per 1,0 gr of the formulation |
| Methyl-p-hydroxybenzoate | 1,50 mg per 1,0 gr of the formulation |
| Methyl salicylate | 0,020 mg per 1,0 gr of the formulation |
| Menthol | 0,030 mg per 1,0 gr of the formulation |
| Water for pharmaceutical use | q.s |
| | |

| Example 5: (Gel 0,5%) | |
|---|---|
| Diclofenac Na | 5,00 mg per 1,0 gr of the formulation |
| Propylene Glycol | 75,00 mg per 1,0 gr of the formulation |
| Methocel E4 MP | 25,00 mg per 1,0 gr of the formulation |
| Methyl-p-hydroxybenzoate | 1,00 mg per 1,0 gr of the formulation |
| Methyl salicylate | 0,040 mg per 1,0 gr of the formulation |
| Menthol | 0,040 mg per 1,0 gr of the formulation |
| Water for pharmaceutical use | q.s |

The invention also relates to a plastic tube comprising a stable semisolid formulation of DiclofenacNa, said device:
Item-1) comprising a blind aluminium tube with internal protective lacquer for pharmaceutical use suitable for containing and disposing semisolids. (Aluminium type 1070 A. EN 573:94)
   The invention also relates to a cap intended for covering the open edge of the tube of item-1, said device:
Item-2) Device comprising of a plastic cap (polyethylene GD 6250) suitable for fitting/screwing to item-1 wherein the cap has a circumference and the tube interfaces with a portion of the circumference of the cap at the tube/cap interface.

The plasmatic levels obtained after the repeated applications of the formulations indicate that the product, even though its high permeation, does not produce plasmatic levels close to the ones achieved through oral administration. This difference gives clinical safety to the preparations.

The present formulation has also an advantageous transdermal action.

The invention is defined by the appended claims.

## Claims

1. Semisolid gel formulation with Diclofenac Na as active substance intended for pharmaceutical use and wherein the gel composition is suitable for the topical, local administration of diclofenac sodium through the skin, said gel comprises diclofenac sodium, and all pharmaceutical acceptable excipients Propylene Glycol, Methocel E4 MP, Methyl-p-hydroxybenzoate, Methyl salicylate, Menthol and purified water for pharmaceutical use.

2. A gel composition according to claim 1 for the topical, local administration of diclofenac sodium through the skin, which comprises diclofenac sodium in a concentration of 0,3 to 10,0 % (w/w) and one or more pharmaceutical acceptable excipients.

3. A gel composition according to any one of claims 1 or 2 wherein the ratio of propylene glycol and methocel lies between 6.0 and 2.0, preferably 4. 0

4. A 1% gel formulation according to anyone or more of the preceding claims 1-3 wherein
| | |
|---|---|
| Diclofenac Na | 10,00 mg per 1,0 gr of the formulation |
| Propylene Glycol | 80,00 mg per 1,0 gr of the formulation |
| Methocel E4 MP | 20,00 mg per 1,0 gr of the formulation |
| Methyl-p-hydroxybenzoate | 1,50 mg per 1,0 gr of the formulation |
| Methyl salicylate | 0,020 mg per 1,0 gr of the formulation |
| Menthol | 0,030 mg per 1,0 gr of the formulation |
| Water for pharmaceutical use | q.s |

5. A 1% gel formulation according to anyone or more of the preceding claims 1-3 wherein
| | |
|---|---|
| Diclofenac Na | 10,00 mg per 1,0 gr of the formulation |
| Propylene Glycol | 70,00 mg per 1,0 gr of the formulation |
| Methocel E4 MP | 30,00 mg per 1,0 gr of the formulation |
| Methyl-p-hydroxybenzoate | 1,50 mg per 1,0 gr of the formulation |
| Methyl salicylate | 0,020 mg per 1,0 gr of the formulation |
| Menthol | 0,030 mg per 1,0 gr of the formulation |
| Water for pharmaceutical use | q.s |

6. A 0,5% gel formulation according to anyone or more of the preceding claims 1-3 wherein
| | |
|---|---|
| Diclofenac Na | 5,00 mg per 1,0 gr of the formulation |
| Propylene Glycol | 70,00 mg per 1,0 gr of the formulation |
| Methocel E4 MP | 30,00 mg per 1,0 gr of the formulation |
| Methyl-p-hydroxybenzoate | 1,50 mg per 1,0 gr of the formulation |
| Methyl salicylate | 0,020 mg per 1,0 gr of the formulation |
| Menthol | 0,030 mg per 1,0 gr of the formulation |
| Water for pharmaceutical use | q.s |

7. A 0,5% gel formulation according to anyone or more of the preceding claims 1-3 wherein
| | |
|---|---|
| Diclofenac Na | 5,00 mg per 1,0 gr of the formulation |
| Propylene Glycol | 80,00 mg per 1,0 gr of the formulation |
| Methocel E4 MP | 20,00 mg per 1,0 gr of the formulation |
| Methyl-p-hydroxybenzoate | 1,50 mg per 1,0 gr of the formulation |
| Methyl salicylate | 0,020 mg per 1,0 gr of the formulation |
| Menthol | 0,030 mg per 1,0 gr of the formulation |
| Water for pharmaceutical use | q.s |

8. A 0,5% gel formulation according to anyone or more of the preceding claims 1-3 wherein
| | |
|---|---|
| Diclofenac Na | 5,00 mg per 1,0 gr of the formulation |
| Propylene Glycol | 75,00 mg per 1,0 gr of the formulation |
| Methocel E4 MP | 25,00 mg per 1,0 gr of the formulation |
| Methyl-p-hydroxybenzoate | 1,00 mg per 1,0 gr of the formulation |
| Methyl salicylate | 0,040 mg per 1,0 gr of the formulation |
| Menthol | 0,040 mg per 1,0 gr of the formulation |
| Water for pharmaceutical use | q.s |

9. Device comprising a blind aluminium tube with internal protective lacquer for pharmaceutical use suitable for containing and disposing semisolids, (Aluminium type 1070 A. EN 573:94) and a plastic cap (polyethylene GD 6250) suitable for fitting/screwing to said tube, wherein the cap has a circumference and the tube interfaces with a portion of the circumference of the cap at the tube/cap interface and wherein it contains a semisolid gel formulation according to claim 1 or anyone of the formulations of claims 2-8.

## Patentansprüche

1. Halbfeste Gel-Formulierung mit Diclofenac-Natrium als aktive Substanz zur pharmazeutischen Verwendung vorgesehen und wobei die Gel-Zusammensetzung für die örtlich begrenzte, lokale Verabreichung von Diclofenac-Natrium durch die Haut geeignet ist, wobei das Gel Diclofenac-Natrium und alle pharmazeutisch akzeptablen Hilfsstoffe Propylenglycol, Methocel E4 MP, Methyl-p-Hydroxybenzoat, Methylsalicylat, Menthol und aufbereitetes wasser zur pharmazeutischen Verwendung enthält.

2. Gel-Zusammensetzung nach Anspruch 1 für die örtlich begrenzte, lokale Verabreichung von Diclofenac-Natrium durch die Haut, welche Diclotenac-Natrium in einer Konzentration von 0.3 bis 10.0% (w/w) und einen order mehrere pharmazeutische Hilfsstoffe enthält.

3. Gel-Zusammensetzung nach einem der Ansprüche 1 oder 2, wobei das Verhältnis von Propylengylcol und Methocel zwischen 6.0 und 2.0, bevorzugt 4.0, liegt.

4. 1%ige Gel-Formulierung nach einem oder mehreren der vorangegangenen Ansprüche 1-3, wobei
| | |
|---|---|
| Diclofenac-Natrium | 10.00 mg pro 1.0 g der Formulierung |
| Propylenglycol | 80.00 mg pro 1.0 g der Formulierung |
| Methocel E4 MP | 20.00 mg pro 1.0 g der Formulierung |
| Methyl-p-hydroxybenzoat | 1.50 mg pro 1.0 g der Formulierung |
| Methylsalicylat | 0.020 mg pro 1.0 g der Formulierung |
| Menthol | 0.030 mg pro 1.0 g der Formulierung |
| Wasser zur pharmazeutischen Verwendung | q.s. |

5. 1%ige Gel-Formulierung nach einem oder mehreren der vorangegangenen Ansprüche 1-3, wobei
| | |
|---|---|
| Diclofenac-Natrium | 10.00 mg pro 1.0 g der Formulierung |
| Propylenglycol | 70.00 mg pro 1.0 g der Formulierung |
| Methocel E4 MP | 30.00 mg pro 1.0 g der Formulierung |
| Methyl-p-hydroxybenzoat | 1.50 mg pro 1.0 g der Formulierung |
| Methylsalicylat | 0.020 mg pro 1.0 g der Formylierung |
| Menthol | 0.030 mg pro 1.0 g der Formulierung |
| Wasser zur pharmazeutischen Verwendung | q.s. |

6. 0.5%ige Gel-Formulierung nach einem oder mehreren der vorangegangenen Ansprüche 1-3, wobei
| | |
|---|---|
| Diclofenac-Natrium | 5.00 mg pro 1.0 g der Formulierung |
| Propylenglycol | 70.00 mg pro 1.0 g der Formulierung |
| Methocel E4 MP | 30.00 mg pro 1.0 g der Formulierung |
| Methyl-p-hydroxybenzoat | 1.50 mg pro 1.0 g der Formulierung |
| Methylsalicylat | 0.020 mg pro 1.0 g der Formulierung |
| Menthol | 0.030 mg pro 1.0 g der Formulierung |
| Wasser zur pharmazeutischen Verwendung | q.s. |

7. 0.5%ige Gel-Formulierung nach einem oder mehreren der vorangegangenen Ansprüche 1-3, wobei
| | |
|---|---|
| Diclofenac-Natrium | 5.00 mg pro 1.0 g der Formulierung |
| Propylenglycol | 80.00 mg pro 1.0 g der Formulierung |
| Methocel E4 MP | 20.00 mg pro 1.0 g der Formulierung |
| Methyl-p-hydroxybenzoat | 1.50 mg pro 1.0 g der Formulierung |
| Methylsalicylat | 0.020 mg pro 1.0 g der Formulierung |
| Menthol | 0.030 mg pro 1.0 g der Formulierung |
| Wasser zur pharmazeutischen Verwendung | q.s. |

8. 0.5%ige Gel-Formulierung nach einem oder mehreren der vorangegangenen Ansprüche 1-3, wobei
| | |
|---|---|
| Diclofenac-Natrium | 5.00 mg pro 1.0 g der Formulierung |
| Propylenglycol | 75.00 mg pro 1.0 g der Formulierung |
| Methocel E4 MP | 25.00 mg pro 1.0 g der Formulierung |
| Methyl-p-hydroxybenzoat | 1.00 mg pro 1.0 g der Formulierung |
| Methylsalicylat | 0.040 mg pro 1.0 g der Formulierung |
| Menthol | 0.040 mg pro 1.0 g der Formulierung |
| Wasser zur pharmazeutischen Verwendung | q.s. |

9. Einrichtung, enthaltend eine Verschluss-Aluminium-Tube mit einem inwendigen Schutzlack zur pharmazeutischen Verwendung, geeignet zum Beinhaltung und Verfügen von fließfähigen Festkörpern (Aluminium Typ 1070 A. EN 573:94) und eine Plastikverschlussklappe (polyethylene GD 6250), geeignet zum Fitting/Verschrauben der Tube, wobei die Verschlusskappe einen Kreisumfang hat und die Tube in einem Bereich des Kreisumfangs der Verschlußkappe an eine Tuben/Verschlusskappen-Übergangsstelle abschließt und wobei sie eine halbfeste Gel-Formulierung nach Anspruch 1 oder eine der Formulierungen nach Ansprüchen 2-8 beinhaltet.

## Revendications

1. Formulation de gel semi-solide avec du diclofénac sodique comme principe actif, destinée à un usage pharmaceutique, et dans laquelle la composition de gel est appropriée pour l'administration locale de diclofénac sodique à travers la peau, ledit gel comprenant du diclofénac sodique et tous les excipients pharmaceutiquement acceptables, propylène-glycol, méthocel E4 MP, méthyl-p-hydroxybenzoate, salicylate de méthyle, menthol et eau purifiée pour usage pharmaceutique.

2. Composition de gel selon la revendication 1, pour l'administration locale de diclofénac sodique à travers la peau, comprenant du diclofénac sodique à une concentration de 0,3 à 10,0 % (p/p) et un ou plusieurs excipients pharmaceutiquement acceptables.

3. Composition de gel selon l'une quelconque des revendications 1 ou 2, dans laquelle le rapport entre le propylène-glycol et le méthocel est compris entre 6,0 et 2,0, de préférence 4,0.

4. Formulation de gel à 1 % selon l'une au moins des revendications 1 à 3, dans laquelle on trouve :
| | |
|---|---|
| Diclofénac sodique | 10,00 mg par 1,0 g de formulation |
| Propylène-glycol | 80,00 mg par 1,0 g de formulation |
| Méthocel E4 MP | 20,00 mg par 1,0 g de formulation |
| Méthyl-p-hydroxybenzoate | 1,50 mg par 1,0 g de formulation |
| Salicylate de méthyle | 0,020 mg par 1,0 g de formulation |
| Menthol | 0,030 mg par 1,0 g de formulation |
| Eau à usage pharmaceutique | q.s |

5. Formulation de gel à 1 % selon l'une au moins des revendications 1 à 3, dans laquelle on trouve :
| | |
|---|---|
| Diclofénac sodique | 10,00 mg par 1,0 g de formulation |
| Propylène-glycol | 70,00 mg par 1,0 g de formulation |
| Méthocel E4 MP | 30,00 mg par 1,0 g de formulation |
| Méthyl-p-hydroxybenzoate | 1,50 mg par 1,0 g de formulation |
| Salicylate de méthyle | 0,020 mg par 1,0 g de formulation |
| Menthol | 0,030 mg par 1,0 g de formulation |
| Eau à usage pharmaceutique | q.s |

6. Formulation de gel à 0,5 % selon l'une au moins des revendications 1 à 3, dans laquelle on trouve :
| | |
|---|---|
| Diclofénac sodique | 5,00 mg par 1,0 g de formulation |
| Propylène-glycol | 70,00 mg par 1,0 g de formulation |
| Méthocel E4 MP | 30,00 mg par 1,0 g de formulation |
| Méthyl-p-hydroxybenzoate | 1,50 mg par 7,0 g de formulation |
| Salicylate de méthyle | 0,020 mg par 1,0 g de formulation |
| Menthol | 0,030 mg par 1,0 g de formulation |
| Eau à usage pharmaceutique | q.s |

7. Formulation de gel à 0,5 % selon l'une au moins des revendications 1 à 3, dans laquelle on trouve :
| | |
|---|---|
| Diclofénac sodique | 5,00 mg par 1,0 g de formulation |
| Propylène-glycol | 80,00 mg par 1,0 g de formulation |
| Méthocel E4 MP | 20,00 mg par 1,0 g de formulation |
| Méthyl-p-hydroxybenzoate | 1,50 mg par 1,0 g de formulation |
| Salicylate de méthyle | 0,020 mg par 1,0 g de formulation |
| Menthol | 0,030 mg par 1,0 g de formulation |
| Eau à usage pharmaceutique | q.s |

8. Formulation de gel à 0,5 % selon l'une au moins des revendications 1 à 3, dans laquelle on trouve :
| | |
|---|---|
| Diclofénac sodique | 5,00 mg par 1,0 g de formulation |
| Propylène-glycol | 75,00 mg par 1,0 g de formulation |
| Méthocel E4 MP | 25,00 mg par 1,0 g de formulation |
| Méthyl-p-hydroxybenzoate | 1,00 mg par 1,0 g de formulation |
| Salicylate de méthyle | 0,040 mg par 1,0 g de formulation |
| Menthol | 0,040 mg par 1,0 g de formulation |
| Eau à usage pharmaceutique | q.s |

9. Dispositif comprenant un tube d'aluminium obturé, avec revêtement de protection interne, à usage pharmaceutique, approprié pour contenir et jeter des préparations semi-solides (Aluminium, type 1070 A. EN 573:94) et un bouchon en matière plastique (polyéthylène GD 6250) conçu pour être adapté/vissé sur ledit tube, dans lequel le bouchon a une circonférence et le tube s'interface avec une partie de la circonférence du bouchon au niveau de l'interface tube/bouchon, lequel contient une formulation de gel semi-solide selon la revendication 1, ou l'une quelconque des formulations des revendications 2 à 8.
